(19) Europäisches Patentamt
European Patent Office
Office européen des brevets

(11) Veröffentlichungsnummer: **0 317 513**
**A2**

(12) # EUROPÄISCHE PATENTANMELDUNG

(21) Anmeldenummer: **88810774.5**

(22) Anmeldetag: **10.11.88**

(51) Int. Cl.[4]: **C 07 D 251/38**
C 07 D 251/46, C 10 M 133/42

(30) Priorität: **19.11.87 CH 4507/87**

(43) Veröffentlichungstag der Anmeldung:
**24.05.89 Patentblatt 89/21**

(84) Benannte Vertragsstaaten: **BE DE FR GB IT**

(71) Anmelder: **CIBA-GEIGY AG**
**Klybeckstrasse 141**
**CH-4002 Basel (CH)**

(72) Erfinder: **Schneider, Rainer, Dr.**
**Am Markstein 4**
**D-6144 Zwingenberg (DE)**

(54) **Mercaptotriazin-Derivate als Schmiermitteladditive.**

(57) Verbindungen der Formel

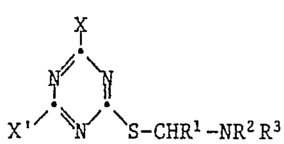

(I)

und der Formel

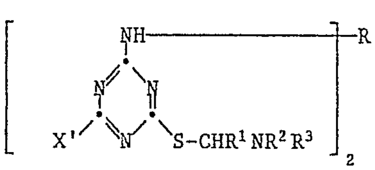

(II),

worin X und X′ beispielsweise -S-CHR$^1$, -NR$^2$R$^3$, -S-R$^3$, -NR$^2$R$^3$ oder -OR$^3$ bedeuten und R$^1$ beispielsweise -H, Alkyl, Cycloalkyl oder Phenyl bedeutet und R$^2$ und R$^3$ beispielsweise -H, Alkyl, Cycloalkyl oder Phenyl bedeutet oder R$^2$ und R$^3$ zusammen einen 3-7-gliedrigen Heterocyclus bilden und R Alkylen ist, und Zusammensetzungen, enthaltend wenigstens eine Verbindung der Formeln I und/oder II und wenigstens einen Schmierstoff, eine Hydraulikflüssigkeit oder eine Metallbearbeitungsflüssigkeit, wobei die Verbindungen der Formel I und/oder II als Hochdruck- und verschleissmindernde Zusätze verwendet werden.

EP 0 317 513 A2

## Beschreibung

### Mercaptotriazin-Derivate als Schmiermitteladditive

Die vorliegende Erfindung betrifft neue Mercaptotriazinverbindungen, diese enthaltende Zusammensetzungen und ihre Verwendung als Schmierstoffadditive.

Es ist bekannt, Schmierstoffen, wie Mineralölen oder synthetischen oder teilsynthetischen Schmierstoffen, Additive zur Verbesserung der Gebrauchseigenschaften zuzusetzen. Insbesondere werden zur Verbesserung der Verschleissschutzeigenschaften den Schmiermitteln Hochdruck- und verschleissmindernde Additive zugefügt. Diese Additive wiederum sollen nicht korrodierend auf die zu schmierenden Metallteile wirken.

Beispielsweise aus der DE-OS-29 21 620 sind phosphor- und schwefelhaltige Verbindungen aus der Reihe der Dialkyldithiophosphate für den genannten Verwendungszweck bekannt geworden. Im Hinblick auf die Verwendung von Katalysatoren in den Abgassystemen von Verbrennungsmotoren ist es ratsam, den Phosphorgehalt in den Schmiermitteln möglichst tief zu halten, damit der Katalysator nicht desaktiviert wird (vergl. H.S. Gandhi et al. Applied Catalysis 3 (1982) 79-88).

Weiter bekannte Verbindungsklassen sind die Zinkdithiophosphate, welche Schmierölen eine verschleissmindernde Wirkung zu verleihen vermögen (Kirk-Othmer, Encyclopedia of Chemical Technology, 3. Auflage, Band 14, Seiten 488 und 492).

Es wurden nun neue Mercaptotriazinderivate gefunden, welche öllöslich und sowohl phosphor- als auch aschefrei sind und die geforderten Eigenschaften bezüglich des Schutzes vor Reibungsabnutzung (Antiwear), Last tragevermögen und Schutz der Metallteile vor Korrosion zu erfüllen vermögen.

Die vorliegende Erfindung betrifft demnach Verbindungen der Formel

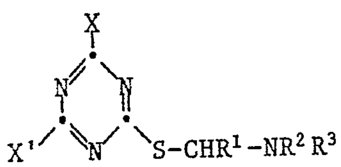

(I),

worin X und X′ gleich oder verschieden sind und $-S-CHR^1-NR^2R^3$, $-S-R^3$, $-NR^2-R^3$ oder $-OR^2$ bedeuten und $R^1$ -H, $C_1$-$C_{12}$-Alkyl, $C_5$-$C_{12}$-Cycloalkyl, 2-Furyl, Phenyl, Naphthyl, $C_7$-$C_{11}$-Aralkyl oder durch $C_1$-$C_4$-Alkyl, $C_1$-$C_4$-Alkoxy, $C_2$-$C_{24}$-Alkoxycarbonyl und/oder Nitro substituiertes Phenyl ist und $R^2$ und $R^3$ gleich oder verschieden sind und -H, $C_1$-$C_{24}$-Alkyl, mit Hydroxy und/oder $C_1$-$C_4$-Alkyl substituiertes $C_1$-$C_{20}$-Alkyl, $C_1$-$C_{20}$-Alkyl, das durch ein oder mehrere -O-, -S- oder -N- unterbrochen ist und/oder Oxo- oder Thionogruppen enthält, $C_3$-$C_{24}$-Alkenyl, $C_3$-$C_{12}$-Alkoxyalkyl, $C_5$-$C_{12}$-Cycloalkyl, $C_7$-$C_{11}$-Aralkyl, unsubstituiertes oder durch $C_1$-$C_{12}$-Alkyl, $C_1$-$C_4$-Alkoxy, $C_2$-$C_{24}$-Alkoxycarbonyl oder Nitro substituiertes Phenyl, Naphthyl, 2-Furyl, 2-Furylmethyl oder 2-(Tetrahydrofuryl)-methyl sind oder worin $R^2$ und $R^3$, zusammen mit dem sie verknüpfenden Stickstoffatom, einen gesättigten oder ungesättigten 3-7-gliedrigen Heterocyclus bilden, oder einen gesättigten oder ungesättigten 3-7-gliedrigen Heterocyclus bilden, welcher weitere Heteroatome der Reihe -O-, -N-, -S-enthält und/oder durch Oxo- oder Thionogruppen substituiert oder durch einen Benzorest anneliert ist, oder worin $R_2$ und $R_3$, zusammen mit dem sie verknüpfenden Stickstoffatom, einen der genannten Heterocyclen bilden, der weiter durch $C_1$-$C_4$-Alkyl, $C_1$-$C_4$-Alkoxy, Amino, Methylamino, $C_1$-$C_4$-Aminoalkyl oder Nitro substituiert ist, und Verbindungen der Formel

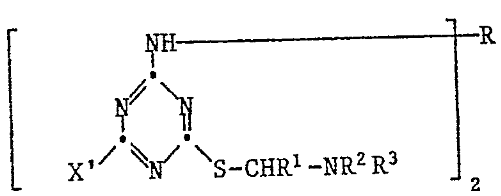

(II),

wobei R $C_1$-$C_{12}$-Alkylen, oder $C_1$-$C_{12}$-Alkylen bedeutet, das durch -O-, -S-oder -N- unterbrochen ist und/oder Oxo- oder Thionogruppen enthält, oder $C_6$-$C_{18}$-Cycloalkylen, $C_6$-$C_{18}$-Arylen, Carbonyl oder Thiocarbonyl bedeutet und X′, $R^1$, $R^2$ und $R^3$ die oben angegebene Bedeutung haben.

$R^1$ kann Alkyl mit 1 bis 12 C-Atomen, $R^2$ und $R^3$ Alkyl mit 1 bis 24 C-Atomen bedeuten.

Beispiele hierfür sind Methyl, Ethyl, n-Propyl, Isopropyl, n-Butyl, sec.-Butyl, t-Butyl, Isoamyl, n-Hexyl, 2-Ethylbutyl, 1-Methylpentyl, 1,3-Dimethylbutyl, 1,1,3,3-Tetramethylbutyl, Isoheptyl, n-Octyl, i-Octyl, 2-Ethylhexyl, 1-Methylheptyl, 1,1,3-Trimethylhexyl, n-Decyl, 1-Methylundecyl oder n-Dodecyl. $R^2$ und $R^3$ als Alkyl können darüber hinaus auch z.B. Tetradecyl, Hexadecyl, Octadecyl oder Eicosyl bedeuten.

Unter $C_5$-$C_{12}$-Cycloalkyl als $R^1$, $R^2$ und $R^3$ sind unsubstituierte oder $C_1$-$C_4$-alkylsubstituierte Cycloalkylre-

ste zu verstehen. Beispiele dafür sind Cyclopentyl, Cyclohexyl, 4-Methylcyclohexyl, Cycloheptyl, Cyclooctyl und Cyclododecyl.

Bedeuten $R^1$, $R^2$ und $R^3$ $C_7$-$C_{11}$-Aralkyl, so handelt es sich dabei insbesondere um Phenyl($C_1$-$C_5$)alkyl oder Naphthylmethyl und beispielsweise um Benzyl, α-Methylbenzyl, α,α-Dimethylbenzyl, 2-Phenylethyl, 1-Phenylethyl oder 2-Phenylpropyl.

$R^1$, $R^2$ und $R^3$ können als substituiertes Phenyl z.B. Tolyl, Xylyl, 4-t-Butylphenyl, 3-Methoxyphenyl, 4-Propoxyphenyl, 3-Nitrophenyl oder 4-Methyl-3-nitrophenyl bedeuten oder können mit Alkoxycarbonyl substituiertes Phenyl sein, wobei Alkoxy z.B. Methoxy, Ethoxy, Isopropoxy oder n-Butoxy sein kann und beispielsweise kann 3-Butoxycarbonylphenyl genannt werden.

$R^2$ und $R^3$ als Alkenyl können z.B. Allyl, Methallyl, 1-Pentenyl, Dodecenyl oder Octadecenyl sein.

$R^2$ und $R^3$ als Alkoxyalkyl können z.B. 2-Methoxyethyl, 2-Isopropoxyethyl, 2-Butoxyethyl oder 2-Octyloxyethyl sein.

Wenn $R^2$ und $R^3$ zusammen mit dem N-Atom einen Heterocyclus bilden, ist dieser vorzugsweise 5- oder 6-gliederig. Falls er noch weitere Heteroatome enthält, ist dieses vorzugsweise ein O- oder N-Atom. Beispiele sind der Pyrrolidin-, Piperidin-, Morpholin-, Thiomorpholin-, Piperazin-, Indol-, Tetrahydrochinolin- oder Tetrahydroisochinolinring.

R als Alkylen kann unverzweigtes oder verzweigtes Alkylen sein, das auch durch -O-, -S- oder -N- unterbrochen sein kann. Beispiele hierfür sind Di-, Tri-, Tetra-, Hexa-, Octa-, Deca- oder Dodecamethylen; 2,2,4-oder 2,4,4-Trimethyl-hexamethylen, 3-Oxa-pentamethylen, 4-Thia-heptamethylen oder 4-(Methylaza)-heptamethylen.

R als $C_6$-$C_{18}$-Cycloalkylen kann eine gesättigte Kohlenwasserstoffgruppe mit zwei freien Valenzen und mindestens einer Ringeinheit sein.

R kann dabei gegebenenfalls auch ein durch $C_1$-$C_4$-Alkyl substituiertes Cycloalkylen darstellen. Beispiele sind: $C_1$-$C_4$-substituiertes Cyclohexylen, wie Methylcyclohexylen, oder 1,4-Decahydronaphthylen.

Ferner soll im vorliegenden Fall unter Cycloalkylen auch Alkylen-cycloalkylen-alkylen mit 8 bis 18 Kohlenstoffatomen, zweckmässig Alkylencyclohexylen-alkylen und beispielsweise Cyclohexylen-1,4-dimethylen, dann Cycloalkylen-alkylen-cycloalkylen mit 13 bis 18 Kohlenstoffatomen, zweckmässig Cyclohexylen-alkylen-cyclohexylen und beispielsweise Dicyclohexylen-methan-4,4-diyl und schliesslich Alkylidendicycloalkylen mit 14 bis 18 Kohlenstoffatomen, zweckmässig Alkylidendicyclohexylen und beispielsweise Isopropylidendicyclohexylen, verstanden werden.

R als Arylen mit 6 bis 18 Kohlenstoffatomen kann eine unsubstituierte oder substituierte aromatische Gruppe darstellen. Zweckmässig enthält R als Arylen 6 bis 10 Kohlenstoffatome und stellt Phenylen, gegebenenfalls substituiert durch $C_1$-$C_4$-Alkyl, dar. Beispielsweise ist R 1,2-Phenylen, 1,3-Phenylen oder 1,4-Phenylen. Mitumfasst von Arylen sollen im vorliegen Fall auch Biphenylen oder Naphthylen sein, die gegebenenfalls durch $C_1$-$C_4$-Alkyl substituiert sind. Als Beispiele dafür lassen sich 1,4-Naphthylen, 4,4′-Diphenylen, Diphenyl-methan-4,4′-diyl oder Diphenyloxid-4,4′-diyl nennen.

Die Summe der in den Resten $R^1$, $R^2$, $R^3$ und R enthaltenen C-Atome beträgt vorzugsweise mehr als 10, insbesondere mehr als 14. Diese Reste tragen zur Löslichkeit in Oel bei.

Bevorzugt sind Verbindungen der Formeln I oder II, worin $R^1$ Wasserstoff ist.

Nach der Erfindung zweckmässige Verbindungen weisen die Formel I auf, worin $R^1$ -H bedeutet und X $-NR^{2'}R^{3'}$ oder $-S-CH_2-NR^2R^3$ und X′ = $-S-CH_2-NR^2R^3$ ist und $R^2$ und $R^3$ unabhängig voneinander -H, $C_1$-$C_{20}$-Alkyl, $C_3$-$C_{24}$-Alkenyl, Cyclohexyl, Phenyl, durch $C_1$-$C_{12}$-Alkyl oder $C_2$-$C_{24}$-Alkoxycarbonyl substituiertes Phenyl, $C_7$-$C_{11}$-Aralkyl oder 3-Methoxy-$C_1$-$C_4$-alkyl bedeuten und $R^{2'}$ und $R^{3'}$ gleich oder verschieden sind und -H, $C_1$-$C_8$-Alkyl, mit Hyxdroxy substituiertes $C_1$-$C_4$-Alkyl, Phenyl oder mit $C_1$-$C_4$-Alkyl substituiertes Phenyl oder $R^{2'}$ und $R^{3'}$ zusammen mit dem sie verknüpfenden Stickstoffatom einen gesättigten 6-gliedrigen Heterocyclus bilden, welcher gegebenenfalls ein Sauerstoffatom enthält, bedeuten.

Zweckmässig sind Verbindungen der Formel II, worin $R^1$ -H bedeutet, X′ $-S-CH_2NR^2R^3$ und $R^2$ und $R^3$ unabhängig voneinander -H, $C_1$-$C_{20}$-Alkyl, $C_3$-$C_{24}$-Alkenyl, Cyclohexyl, Phenyl, durch $C_1$-$C_{12}$-Alkyl oder $C_2$-$C_{24}$-Alkoxycarbonyl substituiertes Phenyl, $C_7$-$C_{11}$-Aralkyl, 2- oder 3-Methoxy-$C_1$-$C_4$-alkyl bedeuten und R $C_1$-$C_{12}$-Alkylen bedeutet.

Bevorzugt werden Verbindungen der Formel I, worin X für $NR^{2'}R^{3'}$ oder $-S-CH_2-N-R^2R^3$ und X′ für $-S-CH_2-R^2R^3$ steht und $R^{2'}$ und $R^{3'}$ gleich oder verschieden sind und -H, $C_1$-$C_4$-Alkyl, vorzugsweise Methyl, $C_1$-$C_4$-Alkyl, das mit Hydroxy substituiert ist, vorzugsweise 2-Hydroxyethyl, oder Phenyl bedeuten oder $R^{2'}$ und $R^{3'}$ zusammen mit dem sie verknüpfenden N-Atom Morpholino bedeuten und $R^2$ und $R^3$ gleich sind und -H, $C_1$-$C_{12}$-Alkyl, mit Hydroxy substituiertes $C_1$-$C_4$-Alkyl, Phenyl oder Cyclohexyl oder $R^2$ und $R^3$ zusammen mit dem sie verknüpfenden N-Atom Morpholino bedeuten.

Bei den Verbindungen der Formel II werden solche bevorzugt, worin X′ für $-S-CH_2-NR^2R^3$ steht, $R^1$ -H ist und $R^2$ und $R^3$ gleich sind und -H, $C_1$-$C_{12}$-Alkyl, mit Hydroxy substituiertes $C_1$-$C_4$-Alkyl, Phenyl oder Cyclohexyl oder $R^2$ und $R^3$ zusammen mit dem sie verknüpfenden N-Atom Morpholino bedeuten.

Weitere ebenfalls bevorzugte Verbindungen sind solche der Formel I, in der X und X′ die Bedeutung von $-S-CH_2-NR^2R^3$ haben und $R^2$ und $R^3$ gleich sind und für $C_4$-$C_8$-Alkyl stehen oder Verbindungen der Formel II, in der X′ die Bedeutung von $-S-CH_2-NR^2R^3$ hat und $R^2$ und $R^3$ gleich sind und für $C_4$-$C_8$-Alkyl stehen und R gleich $C_nH_{2n}$ ist und n eine Zahl von 1 bis 6 bedeutet.

Besonders bevorzugt nach vorliegender Erfindung sind Verbindungen der Formel

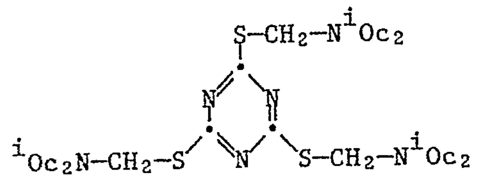 ,

(III)

und

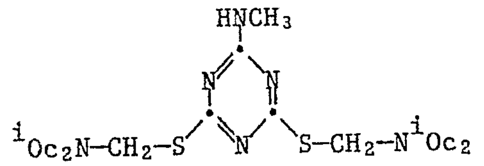 .

(IV)

In vorliegender Anmeldung hat $^{i}$Oc die Bedeutung von 2-Ethylhexyl.

Beispiele bevorzugter Verbindungen nach der Erfindung sind:

2-Amino-4,6-bis-(di-2-ethylhexylamino-methyl-mercapto)-1,3,5-triazin
2-Morpholino-4,6-bis-(di-2-ethylhexylamino-methyl-mercapto)-1,3,5-triazin
2-Phenylamino-4,6-bis-(di-2-ethylhexylamino-methyl-mercapto)-1,3,5-triazin
2-Dihydroxyethylamino-4,6-bis-(di-2-ethylhexylamino-methyl-mercapto)-1,3,5-triazin
2-Methylamino-4,6-bis-(di-2-ethylhexylamino-methyl-mercapto)-1,3,5-triazin
2-Methylamino-4,6-bis-(di-n-butylamino-methyl-mercapto)-1,3,5-triazin
2-Methylamino-4,6-bis-(di-isobutylamino-methyl-mercapto)-1,3,5-triazin
2-Phenylamino-4,6-bis-(di-isobutylamino-methyl-mercapto)-1,3,5-triazin
2-Morpholino-4,6-bis-(di-isobutylamino-methyl-mercapto)-1,3,5-triazin
2,4,6-Tris-(di-isobutylamino-methyl-mercapto)-1,3,5-triazin
2,4,6-Tris-(di-2-ethylhexylamino-methyl-mercapto)-1,3,5-triazin
N,N′-Bis-[4,6-bis(di-2-ethylhexylamino-methyl-mercapto)-1,3,5-triazin-2-yl]-hexamethylen-1,6-diamin
N,N′-Bis-[4,6-bis(di-n-butylamino-methyl-mercapto)-1,3,5-triazin-2-yl]-hexamethylen-1,6-diamin
N,N′-Bis-[4,6-bis(di-isobutylamino-methyl-mercapto)-1,3,5-triazin-2-yl]-hexamethylen-1,6-diamin.

Die Herstellung der Verbindungen nach Formeln I und II kann auf an sich bekannte Weise erfolgen.

Als Beispiel sei ein Herstellungsverfahren erwähnt, gemäss dem ein Mercaptotriazin mit der den zu substituierenden Mercaptogruppen äquivalenten Menge des gewählten Amins und Formaldehyds umgesetzt wird. Nach erfolgter Reaktion können die flüchtigen Bestandteile bei ca. 100°C im Vakuum abdestilliert und das Endprodukte, gegebenenfalls nach einem Klärfiltriervorgang, in hoher Reinheit und Ausbeute gewonnen werden.

Anhand des nachstehend aufgeführten Formelschemas sind in beispielhafter Weise verschiedene Reaktionswege aufgezeichnet für die Umsetzung einer der maximal drei Mercaptogruppen. Analog können die verbleibenden Mercaptogruppen nacheinander mit den gewünschten Resten substituiert werden.

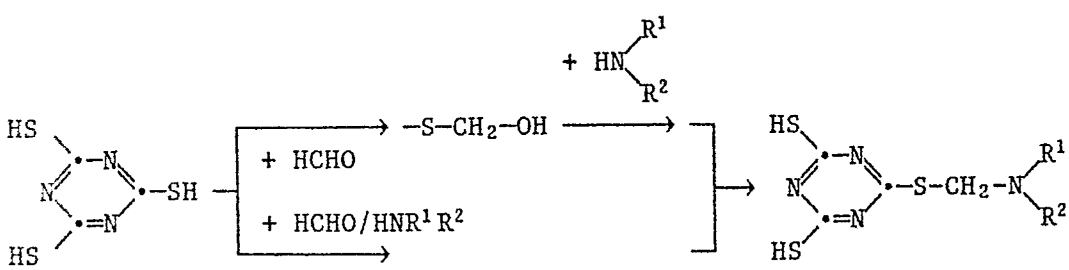


Die erfindungsgemässen Mercaptotriazin-Derivate werden demnach durch eine Mannich-Reaktion aus den entsprechenden Mercaptotriazinen mit Formaldehyd und einem Amin in an sich üblicher Verfahrensweise hergestellt.

Ferner, in einem anderen Herstellungsverfahren, kann entsprechend der an sich bekannten Reaktion am Cyanurchlorid (1,3,5-Trichlor-2,4,6-triazin) bei ca. 0°C, Raumtemperatur und erhöhter Temperatur jeweils ein Chloratom durch die gewünschte Gruppe nach vorliegender Erfindung substituiert werden. Verbindungen der

Formel II beispielsweise können durch Umsetzung von 2 Molen Cyanurchlorid mit einem Mol einer zweiwertigen Verbindung der Formel -NH-R-NH- zum entsprechenden N,N′-Bis[1,3,5-triazin-2-yl]alkylendiamin umgesetzt werden.

Die verbleibenden Chlorgruppen können dann durch die gewünschten Reste, beispielsweise durch -SH Gruppen substituiert werden und daran kann die beschriebene Mannich-Reaktion anschliessen.

Die Verbindungen nach Formel I und Formel II und insbesondere die bevorzugt genannten Verbindungen eignen sich als neue und wertvolle Verschleisschutz- und Hochdruckadditive für sogenannte funktionelle Flüssigkeiten ("functional fluids"), die z.B. Schmierstoffe, wie Schmieröle und -fette, Hydraulikflüssigkeiten und Metallbearbeitungsflüssigkeiten im weitesten Sinne umfassen.

Ein weiterer Gegenstand der Erfindung sind daher auch Zusammensetzungen enthaltend wenigstens eine funktionelle Flüssigkeit im dargelegten Sinne und wenigstens eine Verbindung der Formel I und/oder II.

Die als zweckmässig oder bevorzugt bezeichneten Verbindungen nach vorliegender Erfindung führen bei deren Einsatz in den sog. funktionellen Flüssigkeiten sinngemäss zu zweckmässigen oder bevorzugten Zusammensetzungen.

Der Zusatz der erfindungsgemässen Verbindungen beispielsweise zu Schmierstoffen führt zu einer Verbesserung der allgemeinen Gebrauchseigenschaften, insbesondere der "extreme pressure" und "anti-wear"-Eigenschaften, also der Hochdruck- und Antiverschleisseigenschaften.

Sinngemäss wird auch in Hydraulikflüssigkeiten und Metallbearbeitungsflüssigkeiten eine Verbesserung der allgemeinen Gebrauchseigenschaften erreicht.

Da die Verbindungen keinen Phosphor enthalten, sind sie besonders für Motorenöle geeignet, die in abgasgereinigten Verbrennungs-Motoren Verwendung finden, da eine Schädigung des dem Motor nachgeschalteten Katalysators vermieden werden kann.

Ueberraschenderweise werden auch im FZG-Test (Forschungsstelle Zahnräder Getriebe/TU München, DIN-Norm 51.354) sehr gute Ergebnisse erzielt. Das bedeutet, dass die erfindungsgemässen Verbindungen in Getriebeölen eine herausragende Leistung zeigen.

Dies ist umso überraschender, als es sich um phosphorfreie Verbindungen handelt und den schwefelhaltigen Verbindungen öfters erheblich schlechtere Eigenschaften als den phosphorhaltigen Verbindungen im FZG-Test nachgesagt werden.

Ein weiterer Gegenstand der vorliegenden Erfindung ist deshalb die Verwendung von Verbindungen der Formeln I und II, und bevorzugt der Formeln III oder IV, als Hochdruck- und verschleissmindernde Zusätze zu Schmierstoffen, als Zusätze zu Hydraulikflüssigkeiten und als Zusätze zu Metallbearbeitungsflüssigkeiten.

Die Verbindungen der Formeln I und II werden den Schmierstoffen, Hydraulikflüssigkeiten oder Metallbearbeitungsflüssigkeiten zweckmässig in einer Menge von 0,05 bis 5 Gew.-%, bevorzugt in einer Menge von 0,1 bis 3 Gew.-%, bezogen auf das Gesamtgewicht der Schmierstoffe, Hydraulikflüssigkeien oder Metallbearbeitungsflüssigkeiten zugesetzt.

Die in Frage kommenden Schmierstoffe, Hydraulikflüssigkeiten und Metallbearbeitungsflüssigkeiten sind dem Fachmann geläufig und z.B. im "Schmiermittel Taschenbuch" (Hüthig Verlag, Heidelberg, 1974) resp. in "Ullmanns Encyclopädie der technischen Chemie" Bd. 13, Seiten 85-94 (Verlag Chemie, Weinheim, 1977) oder von D. Klamann in "Schmierstoffe und verwandte Produkte" Verlag Chemie, Weinheim (1982) beschrieben.

Der Schmierstoff kann beispielsweise ein Oel, basierend auf einem Mineralöl oder einem synthetischen Oel, oder ein Gemisch aus mineralischem Oel und synthetischem Oel oder ein Fett sein. Der Ausdruck Mineralöl umfasst alle Mineralöle für Schmierzwecke, wie Mineralöle auf Kohlenwasserstoffbasis. Synthetische Oele können beispielsweise aliphatische oder aromatische Carboxylester, polymere Ester, Polyalkylenoxide, Phosphorsäureester, Poly-α-olefine, Silicone, Glykole, Polyglykole oder Polyalkylenglykole sein.

Die Schmierstoffe oder Hydraulikflüssigkeiten können zusätzlich andere Additive enthalten, die zugegeben werden, um die Grundeigenschaften von Schmierstoffen oder allgemein der "functional fluids" noch weiter zu verbessern; dazu gehören Antioxidantien, Metallpassivatoren, Rostinhibitoren, Viskositätsindex-Verbesserer, Stockpunkterniedriger, Dispergiermittel, Detergentien, weitere Hochdruck-Zusätze sowie andere Verschleissschutz-Additive.

Beispiele für phenolische Antioxidantien

1. Alkylierte Monophenole

2,6-Di-tert-butyl-4-methylphenol, 2,6-Di-tert-butylphenol, 2-tert-Butyl-4,6-dimethylphenol, 2,6-Di-tert-butyl-4-ethylphenol, 2,6-Di-tert-butyl-4-n-butylphenol, 2,6-Di-tert-butyl-4-iso-butylphenol, 2,6-Di-cyclopentyl-4-methylphenol, 2-(α-Methylcyclohexyl)-4,6-dimethylphenol,2,6-Di-octadecyl-4-methylphenol, 2,4,6-Tri-cyclohexylphenol, 2,6-Di-tert-butyl-4-methoxymethylphenol, o-tert-Butylphenol.

2. Alkylierte Hydrochinone

2,6-Di-tert-butyl-4-methoxyphenol, 2,5-Di-tert-butyl-hydrochinon, 2,5-Di-tert-amyl-hydrochinon, 2,6-Diphenyl-4-octadecyloxyphenol.

3. Hydroxylierte Thiodiphenylether

2,2′-Thio-bis-(6-tert-butyl-4-methylphenol), 2,2′-Thio-bis-(4-octylphenol), 4,4′-Thio-bis-(6-tert-butyl-3-methylphenol), 4,4′-Thio-bis-(6-tert-butyl-2-methylphenol).

4. Alkyliden-Bisphenole
2,2′-Methylen-bis-(6-tert-butyl-4-methylphenol), 2,2′-Methylen-bis-(6-tert-butyl-4-ethylphenol), 2,2′-Methylen-bis-[4-methyl-6-(α-methylcyclohexyl)-phenol], 2,2′-Methylen-bis-(4-methyl-6-cyclohexylphenol), 2,2′-Methylen-bis-(6-nonyl-4-methylphenol), 2,2′-Methylen-bis-(4,6-di-tert-butylphenol), 2,2′-Ethyliden-bis-(4,6-di-tert-butylphenol), 2,2′-Ethyliden-bis-(6-tert-butyl-4-iso-butylphenol), 2,2′-Methylen-bis-[6-(α-methylbenzyl)-4-nonylphenol], 2,2′-Methylen-bis-[6-(α,α-dimethylbenzyl)-4-nonylphenol], 4,4′-Methylen-bis-(2,6-di-tert-butylphenol), 4,4′-Methylen-bis-(6-tert-butyl-2-methylphenol), 1,1-Bis-(5-tert-butyl-4-hydroxy-2-methylphenyl)-butan, 2,6-Di-(3-tert-butyl-5-methyl-2-hydroxybenzyl)-4-methylphenol, 1,1,3-Tris-(5-tert-butyl-4-hydroxy-2-methylphenyl)-3-n-dodecylmercaptobutan, Ethylenglycol-bis-[3,3-bis-(3′-tert-butyl-4′-hydroxyphenyl)-butyrat], Bis-(3-tert-butyl-4-hydroxy-4-methylphenyl)-dicyclopentadien, Bis-[2-(3′-tert-butyl-2′-hydroxy-5′-methylbenzyl)-6-tert-butyl-4methyl-phenyl]-terephthalat.

5.Benzylverbindungen
1,3,5-Tri-(3,5-di-tert-butyl-4-hydroxybenzyl)-2,4,6-trimethylbenzol, Bis-(3,5-di-tert-butyl-4-hydroxybenzyl)-sulfid, 3,5-Di-tert-butyl-4-hydroxybenzyl-mercaptoessigsäure-isooctylester, Bis-(4-tert-butyl-3-hydroxy-2,6-dimethylbenzyl)-dithiol-terephthalat, 1,3,5-Tris-(3,5-di-tert-butyl-4-hydroxybenzyl)-isocyanurat, 1,3,5-Tris-(4-tert-butyl-3-hydroxy-2,6-dimethylbenzyl)-isocyanurat, 3,5-Di-tert-butyl-4-hydroxybenzyl-phosphonsäure-dioctadecylester, 3,5-Di-tert-butyl-4-hydroxybenzylphosphonsäure-monoethylester, Calcium-salz.

6. Acylaminophenole
4-Hydroxy-laurinsäureanilid, 4-Hydroxy-stearinsäureanilid, 2,4-Bis-octylmercapto-6-(3,5-di-tert-butyl-4-hydroxyanilino)-s-triazin, N-(3,5-di-tert-butyl-4-hydroxyphenyl)-carbaminsäureoctylester.

7. Ester der β-(3,5-Di-tert-butyl-4-hydroxyphenyl)-propionsäure
mit ein- oder mehrwertigen Alkoholen, wie z.B. mit
Methanol, Diethylenglycol, Octadecanol, Triethylenglycol, 1,6-Hexandiol, Pentaerythrit, Neopentylglycol, Tris-hydroxyethyl-isocyanurat, Thiodiethylenglycol, Bis-hydroxyethyl-oxalsäurediamid.

8. Ester der β-(5-tert-butyl-4-hydroxy-3-methylphenyl)-propionsäure
mit ein- oder mehrwertigen Alkoholen, wie z.B. mit
Methanol, Diethylenglycol, Octadecanol, Triethylenglycol, 1,6-Hexandiol, Pentaerythrit, Neopentylglycol, Tris-hydroxyethyl-isocyanurat, Thiodiethylenglycol, Di-hydroxyethyl-oxalsäurediamid.

9. Amide der β-(3,5-Di-tert-butyl-4-hydroxyphenyl)-propionsäure,
wie z.B.
N,N′-Bis-(3,5-di-tert-butyl-4-hydroxyphenylpropionyl)-hexamethylendiamin, N,N′-Bis-(3,5-di-tert-butyl-4-hydroxyphenylpropionyl)-trimethylendiamin, N,N′-Bis-(3,5-di-tert-butyl-4-hydroxyphenylpropionyl)-hydrazin.

Beispiele für aminische Antioxidantien:
N.N′-Di-isopropyl-p-phenylendiamin, N,N′-Di-sec-butyl-p-phenylendiamin, N,N′-Bis(1,4-dimethyl-pentyl)-p-phenylendiamin, N,N′-Bis(1-ethyl-3-methyl-pentyl)-p-phenylendiamin, N,N′-Bis(1-methyl-heptyl)-p-phenylendiamin, N,N′-Diphenyl-p-phenylendiamin, N,N′-Di-(naphthyl-2)-p-phenylendiamin, N-Isopropyl-N′-phenyl-p-phenylendiamin, N-(1,3-Dimethyl-butyl)-N′-phenyl-p-phenylendiamin, N-(1-Methyl-heptyl)-N′-phenyl-p-phenylendiamin, N-Cyclohexyl-N′-phenyl-p-phenylendiamin, 4-(p-Toluol-sulfonamido)-di-phenylamin, N,N′-Dimethyl-N,N′-di-sec-butyl-p-phenylendiamin, Diphenylamin, N-Allyldiphenylamin, 4-Isopropoxy-diphenylamin, N-Phenyl-1-naphthylamin, N-Phenyl-2-naphthylamin, octyliertes Diphenylamin, z.B. p,p′-di-tert-Octyldiphenylamin, 4-n-Butylaminophenol, 4-Butyrylamino-phenol, 4-Nonanoylamino-phenol, 4-Dodecanoylamino-phenol, 4-Octadecanoylaminophenol, Di-(4-methoxy-phenyl)-amin, 2,6-Di-tert-butyl-4-dimethylaminomethyl-phenol, 2,4′-Diamino-diphenylmethan, 4,4′-Diamino-diphenylmethan, N,N,N′,N′-Tetramethyl-4,4′-diamino-diphenylmethan, 1,2-Di-[(2-methyl-phenyl)-amino]-ethan, 1,2-Di-(phenylamino)-propan, (o-Tolyl)-bi-guanid, Di-[4-(1′,3′-dimethyl-butyl)-phenyl)amin, tert-octyliertes N-Phenyl-1-naphthylamin, Gemisch aus mono- und dialkylierten tert-Butyl-/tert-Octyldiphenylaminen, 2,3-Dihydro-3,3-dimethyl-4H-1,4-benzothiazin, Phenothiazin, N-Allylphenothiazin.

Beispiele für weitere Antioxidantien
Aliphatische oder aromatische Phosphite, Ester der Thiodipropionsäure oder der Thiodiessigsäure, oder Salze der Dithiocarbamid- oder Dithiophosphorsäure.

Beispiele für Metallpassivatoren sind:
für Kupfer, z.B.:
Triazole, Benztriazole und deren Derivate, 2-Mercaptobenzthiazol, 5,5′-Methylenbisbenztriazol, 4,5,6,7-Tetrahydrobenztriazol, 2,5-Di-mercaptothiadiazol, Salicyliden-propylendiamin, Salze von Salicylaminoguanidin.

Beispiele für Rost-Inhibitoren sind:

a) Organische Säuren, ihre Ester, Metallsalze und Anhydride, z.B.:
N-Oleoyl-sarcosin, Sorbitan-mono-oleat, Blei-naphthenat, Dodecenylbernsteinsäure-anhydrid, Alkenylbernsteinsäure-Halbester, 4-Nonylphenoxy-essigsäure.
b) Stickstoffhaltige Verbindungen, z.B.:
I. Primäre, sekundäre oder tertiäre aliphatische oder cycloaliphatische Amine und Amin-Salze von organischen und anorganischen Säuren, z.B. öllössliche Alkylammoniumcarboxylate.
II. Heterocyclische Verbindungen, z.B.:
Substituierte Imidazoline und Oxazoline.
c) Phosphorhaltige Verbindungen, z.B.:
Aminsalze von Phosphorsäurepartialestern.
d) Schwefelhaltige Verbindungen, z.B.:
Barium-dinonylnaphthalin-sulfonate, Calciumpetroleum-sulfonate.

Beispiele für Viskositätsindex-Verbesserer sind:
Polyacrylate, Polymethacrylate, Vinylpyrrolidon/Methacrylat-Copolymere, Polyvinylpyrrolidone, Polybutene, Olefin-Copolymere, Styrol/Acrylat-Copolymere, Polyether.

Beispiele für Stockpunkterniedriger sind:
Polymethacrylat, alkylierte Naphthalinderivate.

Beispiele für Dispergiermittel/Tenside sind:
Polybutenylbernsteinsäure-imide, Polybutenylphosphonsäurederivate, basische Magnesium-, Calcium-, und Bariumsulfonate und -phenolate.

Beispiele für Verschleissschutz-Additive sind:
Schwefel und/oder Phosphor und/oder Halogen enthaltende Verbindungen, wie geschwefelte pflanzliche Oele, Zinkdialkyldithiophosphate, Tritolylphosphat, chlorierte Paraffine, Alkyl- und Aryldisulfide, Triphenylphosphorothionate, Diethanolaminomethyltolyltriazol und Di(2-isooctyl)aminomethyltolyltriazol.

Die erfindungsgemässen Schmierstoffzusammensetzungen können auch ein Co-Schmiersystem, beispielsweise durch Zusatz von üblichen Mengen von Festschmierstoffen, wie Bornitrid, Graphit, Molybdändisulfid, Teflon (Tetrafluorethylen) usw., enthalten.

Die Erfindung ist anhand nachfolgender Beispiele noch weiter erläutert.

Die Angaben in Teilen und Prozenten beziehen sich auf das Gewicht, sofern nichts anderes angegeben ist.

Beispiel 1: 2-Methylamino-4,6-bis-(di-2-ethylhexylamino-methylmercapto)-1,3,5-triazin

1 Mol (174 g) 2-Methylamino-4,6-bis-mercapto-1,3,5-triazin wird mit 2 Mol (483 g) Di-2-ethylhexylamin und mit 162 g einer37%igen Formaldehydlösung so versetzt, dass die Temperatur auf ca. 70°C steigt. Bei dieser Temperatur wird die Mischung für 1 Stunde gerührt. Anschliessend werden die flüchtigen Bestandteile bei 100°C und < 70 mbar abdestilliert und das Endprodukt klärfiltriert. Man erhält die gewünschte Verbindung als gelbe, zähe Flüssigkeit mit einem $n_D^{20}$ von 1,5102.

Beispiel 2: 2,4,6-Tris-(di-2-ethylhexylamino-methyl-mercapto)-1,3,5-triazin

1 Mol (177 g) Triosmercaptotriazin wird mit 3 Mol (724,5 g) Di-2-äthylhexylamin und mit 243 g einer 37%igen Formaldehydlösung so versetzt, dass die Temperatur auf ca. 70°C steigt. Bei dieser Temperatur wird die Mischung für 1 Stunde gerührt. Anschliessend werden die flüchtigen Bestandteile bei 100°C und < 70 mbar abdestilliert und das Endprodukt klärfiltriert.
Man erhält die gewünschte Verbindung als gelbe Flüssigkeit mit einem $n_D^{20}$ von 1,5050

Beispiele 3-11:

In Analogie zu den Beispielen 1 und 2 werden die in Tabelle 1 angegebenen Verbindungen erhalten.

Tabelle 1

| Beispiel | 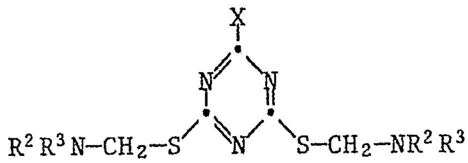 | | $n_D^{20}$ |
|---|---|---|---|
| 3 | $NR^2R^3 = N^{i}Oc_2$ 1) | $X = -NH_2$ | 1,5147 |
| 4 | $= N^{i}Oc_2$ 1) | = 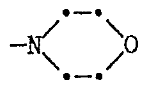 | 1,5221 |
| 5 | $= N^{i}Oc_2$ 1) | = –NHPh | 1,5342 |
| 6 | $= N^{i}Oc_2$ 1) | $= -N(C_2H_4OH)_2$ | 1,4974 |
| 7 | $= -N^{n}Bu_2$ | $= -NHCH_3$ | 1,5380 |
| 8 | $= -N^{i}Bu_2$ | $= -NHCH_3$ | 1,5262 |
| 9 | $= -N^{i}Bu_2$ | = –NHPh | 1,5540 |
| 10 | $= -N^{i}Bu_2$ | = 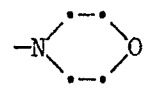 | Fp. 88°C |
| 11 | $= -N^{i}Bu_2$ | $= -S-CH_2-N^{i}Bu_2$ | 1,5109 |

Tabelle 1 (Fortsetzung)

| Beispiel | 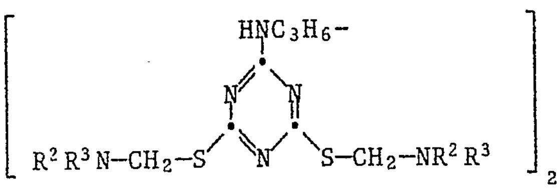 | $n_D^{20}$ |
|---|---|---|
| 12 | $NR^2R^3 = N^{i}Oc_2$ 1) | 1,5159 |
| 13 | $= N^{i}Bu_2$ | 1,5302 |
| 14 | $= N^{n}Bu_2$ | 1,5152 |

1) $^{i}Oc$ = 2-äthylhexyl

Zur Prüfung der synthetisierten Verbindungen werden die folgenden Prüfverfahren herangezogen:

Mit dem Shell-Vierkugel Apparat (IP 239/73 Extreme pressure und wear lubricant test for oils and greases - four ball machine; ASTM-D 2783-81) werden folgende Werte bestimmt:

1. W.L. = Weld load (Schweisslast in (N)).

Gibt die Last an, bei der die 4 Kugeln innerhalb von 10 Sekunden zusammenschweissen.

2. W.S.D. = Wear Scar Diameter in (mm). Mittlerer Verschleissdurchmesser bei einer Belastung von 40 kg während 1 h.

Als Basisöl wird SSU 150 der Mobil verwendet. Die erfindungsgemässe jeweilige Verbindung wird in einer Konzentration von 1 Gew.-% zugesetzt.

Die Resultate sind in Tabelle 2 wiedergegeben.

Tabelle 2

| Additive aus Beispiel | 1. W.L. (N) | 2. W.S.D. (mm) |
|---|---|---|
| Basisöl | 1200 | 0,9 |
| Beispiel 1 | 1600 | 0,5 |
| Beispiel 2 | 1800 | 0,7 |
| Beispiel 3 | 1600 | 0,5 |
| Beispiel 4 | 1600 | 0,5 |
| Beispiel 5 | 1800 | 0,55 |
| Beispiel 8 | 1800 | 0,55 |
| Beispiel 9 | 1600 | 0,55 |
| Beispiel 11 | 1800 | 0,65 |
| Beispiel 13 | 1600 | 0,55 |
| Beispiel 14 | 1800 | 0,55 |

Die Ergebnisse aus dem FZG-Test sind in Tabelle 3 aufgeführt. (Forschungsstelle Zahnräder Getriebe / TU München, DIN-Norm 51.354)

Dabei wird eine Zusammensetzung aus 0,25 % der erfindungsgemässen Verbindung in einem Grundöl des Types Shell Catenex P 941 geprüft und die "failure load stage" (FLS) oder Schadenslaststufe bestimmt. Die ermittelten Werte lassen sich aus Tabelle 3 entnehmen.

Tabelle 3

| Additiv aus Beispiel | (0,25 % in Catenex P 941) FLS |
|---|---|
| Basisöl | 6 |
| Beispiel 1 | >12 |
| Beispiel 2 | >12 |
| Beispiel 8 | 10 |

**Patentansprüche**

1. Verbindungen der Formel

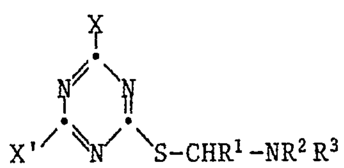

(I),

worin X und X′ gleich oder verschieden sind und $-S-CHR^1-NR^2R^3$, $-S-R^3$, $-NR^2R^3$ oder $-OR^2$ bedeuten und $R^1$ -H, $C_1$-$C_{12}$-Alkyl, $C_5$-$C_{12}$-Cycloalkyl, 2-Furyl, Phenyl, Naphthyl, $C_7$-$C_{11}$-Aralkyl oder durch $C_1$-$C_4$-Alkyl, $C_1$-$C_4$-Alkoxy, $C_2$-$C_{24}$-Alkoxycarbonyl und/oder Nitro substituiertes Phenyl ist und $R^2$ und $R^3$ gleich oder verschieden sind und -H, $C_1$-$C_{24}$-Alkyl, mit Hydroxy und/oder $C_1$-$C_4$-Alkyl substituiertes $C_1$-$C_{20}$-Alkyl, $C_1$-$C_{20}$-Alkyl, das durch ein oder mehrere -O-, -S- oder -N- unterbrochen ist und/oder Oxo-

oder Thionogruppen enthält, $C_3$-$C_{24}$-Alkenyl, $C_3$-$C_{12}$-Alkoxyalkyl, $C_5$-$C_{12}$-Cycloalkyl, $C_7$-$C_{11}$-Aralkyl, unsubstituiertes oder durch $C_1$-$C_{12}$-Alkyl, $C_1$-$C_4$-Alkoxy, $C_2$-$C_{24}$-Alkoxycarbonyl oder Nitro substituiertes Phenyl, Naphthyl, 2-Furyl, 2-Furylmethyl oder 2-(Tetrahydrofuryl)-methyl sind oder worin $R^2$ und $R^3$, zusammen mit dem sie verknüpfenden Stickstoffatom, einen gesättigten oder ungesättigten 3-7-gliedrigen Heterocyclus bilden, oder einen gesättigten oder ungesättigten 3-7-gliedrigen Heterocyclus bilden welcher weitere Heteroatome der Reihe -O-, -N-, -S-enthält und/oder durch Oxo- oder Thionogruppen substituiert oder durch einen Benzorest anneliert ist, oder worin $R^2$ und $R^3$, zusammen mit dem sie verknüpfenden Stickstoffatom, einen der genannten Heterocyclen bilden, der weiter durch $C_1$-$C_4$-Alkyl, $C_1$-$C_4$-Alkoxy, Amino, Methylamino, $C_1$-$C_4$-Aminoalkyl oder Nitro substituiert ist, und Verbindungen der Formel

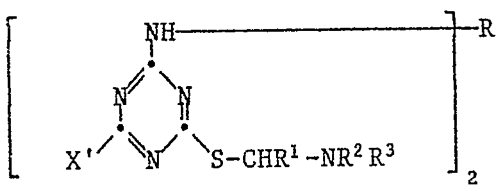

(II),

wobei R $C_1$-$C_{12}$-Alkylen oder $C_1$-$C_{12}$-Alkylen bedeutet, das durch -O-, -S-oder -N- unterbrochen ist und/oder Oxo- oder Thionogruppen enthält, oder $C_6$-$C_{18}$-Cycloalkylen, $C_6$-$C_{18}$-Arylen, Carbonyl oder Thiocarbonyl bedeutet und X′, $R^1$, $R^2$ und $R^3$ die oben angegebene Bedeutung haben.

2. Verbindungen der Formel I nach Anspruch 1, worin $R^1$ -H bedeutet, X $-NR^{2\prime}R^{3\prime}$ oder $-S-CH_2-NR^2R^3$ bedeutet und X′ $-S-CH_2-NR^2R^3$ bedeutet, und $R^2$ und $R^3$ unabhängig voneinander -H, $C_1$-$C_{20}$-Alkyl, $C_3$-$C_{24}$-Alkenyl, Cyclohexyl, Phenyl, durch $C_1$-$C_{12}$-Alkyl oder $C_2$-$C_{24}$-Alkoxycarbonyl substituiertes Phenyl, $C_7$-$C_{11}$-Aralkyl, 2- oder 3-Methoxy-$C_1$-$C_4$-alkyl bedeuten und $R^{2\prime}$ und $R^{3\prime}$ gleich oder verschieden sind und -H, $C_1$-$C_8$-Alkyl, mit Hydroxy substiutiertes $C_1$-$C_4$-Alkyl, Phenyl oder mit $C_1$-$C_4$-Alkyl substituiertes Phenyl oder $R^{2\prime}$ und $R^{3\prime}$ zusammen mit dem sie verknüpfenden Stickstoffatom einen gesättigten 6-gliedrigen Heterocyclus bilden, oder einen gesättigten 6-gliedrigen Heterocyclus bilden, welcher ein Sauerstoffatom enthält.

3. Verbindungen der Formel II nach Anspruch 1, worin $R^1$ -H bedeutet, X′ $-S-CH_2-NR^2R^3$ und $R^2$ und $R^3$ unabhängig voneinander -H, $C_1$-$C_{20}$-Alkyl, $C_3$-$C_{24}$-Alkenyl, Cyclohexyl, Phenyl, durch $C_1$-$C_{12}$-Alkyl oder $C_2$-$C_{24}$-Alkoxycarbonyl substituiertes Phenyl, $C_7$-$C_{11}$-Aralkyl, 2- oder 3-Methoxy-$C_1$-$C_4$-alkyl bedeuten und R $C_1$-$C_{12}$-Alkylen bedeutet.

4. Verbindungen der Formel I nach Anspruch 1, worin X für $NR^{2\prime}R^{3\prime}$ oder $-S-CH_2-NR^2R^3$ und X′ für $-S-CH_2-NR^2R^3$ steht und $R^{2\prime}$ und $R^{3\prime}$ gleich oder verschieden sind und -H, $C_1$-$C_4$-Alkyl, vorzugsweise Methyl, $C_1$-$C_4$-Alkyl, das mit Hydroxy substituiert ist, vorzugsweise 2-Hydroxyethyl oder Phenyl bedeuten oder $R^{2\prime}$ und $R^{3\prime}$ zusammen mit dem sie verknüpfenden N-Atom Morpholino bedeuten und $R^2$ und $R^3$ gleich sind und -H,$C_1$-$C_{12}$-Alkyl, mit Hydroxy substituiertes $C_1$-$C_4$-Alkyl, Phenyl oder Cyclohexyl oder $R^2$ und $R^3$ zusammen mit dem sie verknüpfenden N-Atom Morpholino bedeuten.

5. Verbindungen der Formel II nach Anspruch 1, worin X′ für $-S-CH_2-NR^2R^3$ steht, $R^1$ -H ist und $R^2$ und $R^3$ gleich sind und -H, $C_1$-$C_{12}$-Alkyl, mit Hydroxy substituiertes $C_1$-$C_4$-Alkyl, Phenyl oder Cyclohexyl oder $R^2$ und $R^3$ zusammen mit dem sie verknüpfenden N-Atom Morpholino bedeuten.

6. Verbindungen der Formel I nach Anspruch 1, in der X und X′ die Bedeutung von $-S-CH_2-NR^2R^3$ haben und $R^2$ und $R^3$ gleich sind und für $C_4$-$C_8$-Alkyl stehen oder Verbindungen der Formel II nach Anspruch 1, in der X′ die Bedeutung von $-S-CH_2-NR^2R^3$ hat und $R^2$ und $R^3$ gleich sind und für $C_4$-$C_8$-Alkyl stehen und R gleich $C_nH_{2n}$ ist und n eine Zahl von 1 bis 6 bedeutet.

7. Verbindungen nach Anspruch 1 der Formeln

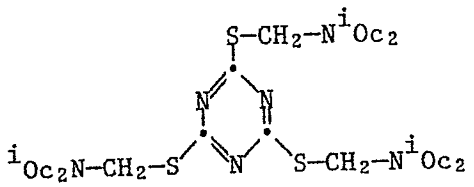

(III)

,

und

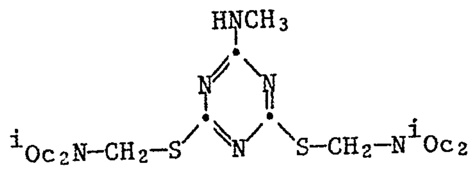

(IV)

,

wobei ⁱOc für 2-Ethylhexyl steht.

8. Zusammensetzung, enthaltend

a) mindestens einen Schmierstoff oder mindestens eine Hydraulikflüssigkeit oder mindestens eine Metallbearbeitungsflüssigkeit und

b) mindestens eine Verbindung der Formel I und/oder der Formel II nach Anspruch 1.

9. Zusammensetzung nach Anspruch 8, enthaltend 0,05 bis 5 Gewichts-%, vorzugsweise 0,1 bis 3 Gew.-%, bezogen auf den Schmierstoff, die Hydraulikflüssigkeit oder Metallbearbeitungsflüssigkeit mindestens einer Verbindung der Formel I und/oder der Formel II nach Anspruch 1.

10. Verwendung der Verbindungen der Formel I und der Formel II gemäss Anspruch 1 als Hochdruck- und verschleissmindernde Zusätze in Schmierstoffen, in Hydraulikflüssigkeiten und in Metallbearbeitungsflüssigkeiten.